# EUROPEAN PATENT SPECIFICATION

(11) **EP 2 228 436 B1**
(45) Date of publication and mention of the grant of the patent: **13.01.2016**
(21) Application number: 10155651.2
(22) Date of filing: 05.03.2010
(51) Int. Cl.: C12N 1/00, C12Q 1/24, G01N 33/569, G01N 1/40, C12N 1/02

(54) **Centrifugation and filtration methods for concentrating microorganisms**
Zentrifugations- und Filterverfahren zum Konzentrieren von Mikroorganismen
Procédés de centrifugation et de filtration pour concentrer des micro-organismes

(30) Priority: 06.03.2009 US 157945 P
(43) Date of publication of application: 15.09.2010
(73) Proprietor: DuPont Nutrition Biosciences ApS, 1001 Copenhagen K. (DK)
(72) Inventor: Petersen, Lars Wexoe, Muskego, WI 53150 (US); Hohol, Eric, Stoughton, WI 53589 (US); Von Dollen, Steve E., Madison, WI 53714 (US)
(74) Representative: Cabinet Plasseraud

(56) References cited:
- WO-A2-2009/085357
- US-A- 5 961 846
- BERNHARDT MATHIAS ET AL: "detection of bacteria in blood by centrifugation and filtration" JOURNAL OF CLINNICAL MICROBIOLOGY, vol. 29, no. 3, March 1991 (1991-03), pages 422-425, XP002577620

## Description

### Field of the Invention

The present invention provides processes for concentrating microorganism-containing suspensions. In some preferred embodiments, the processes comprise a centrifugation step followed by a filtration step. This process is particularly suitable for concentrating microorganism-containing suspensions (*e*.*g*., lactic acid bacteria suspensions) on an industrial scale.

### Background of the Invention

Before inoculation into products, microorganisms are cultured in order to provide a suspension containing large amounts of microorganisms. The suspension is then usually concentrated using a single concentration step such as centrifugation, filtration, distillation, sedimentation or flocculation. This concentration step is often followed freezing or freeze-drying or storage of the microbial suspension in liquid nitrogen to preserve and/or store the microorganisms. In addition to providing a suspension of desired microorganisms, the concentration step also reduces the volume of the suspension to be treated for preservation and/or storage. By reducing the volume of the suspension and increasing the concentration of the microorganisms in the suspension cost reduction advantages are obtained.

The concentration step is very important on industrial scale processes, as efficient concentration of microbial suspensions is needed in order to reduce the costs associated with culture preservation, storage and transport. As methods such as filtration are not usually economically feasible, centrifugation is traditionally used for concentration of microorganism suspensions at industrial scale. However, pressure filtration has found some use. In addition, some suspensions are difficult to efficiently concentrate and produce sufficient concentrations of organisms in the final suspension. Use of high gravitational force has been used to increase concentration.

In addition, centrifugation and filtration both have their limits, especially when applied to industrial scale production. Centrifugation provides a limited increase in microorganism concentration. In addition, at industrial scale, the concentrate must remain sufficiently flowable to permit further processing of solution. Thus, the microbial activity and concentration is limited by the relative density of the organisms and medium used. Filtration of large volumes is very expensive. In addition, the dead space in the large filtration areas required results in some loss of microorganisms during the filtration process.

Therefore, there is still a need to improve the efficiency of concentration methods suitable for concentrating microorganism-containing suspensions with a high concentration rate and a limited loss of activity (*i*.*e*., a limited loss of viable microorganisms). These methods need to be feasible at any scale, but especially on the industrial scale, where large volumes of suspension are concentrated.

### Summary of the Invention

The present invention provides processes for concentrating microorganism-containing suspensions. In some preferred embodiments, the processes comprise a centrifugation step followed by a filtration step. This process is particularly suitable for concentrating microorganism-containing suspensions (*e*.*g*., lactic acid bacteria suspensions) on an industrial scale.

The present invention provides processes comprising the following steps: (a) centrifuging a microorganism-containing suspension to provide a first microorganism-containing concentrate and a supernatant liquid; (b) filtering the first microorganism-containing concentrate, to provide a permeate and a second microorganism-containing concentrate.

In some embodiments, the process is a continuous flow process comprising: (a₁) centrifuging a microorganism-containing suspension to provide a first microorganism-containing concentrate and a supernatant liquid; (a₂) continuously withdrawing the first microorganism-containing concentrate during centrifugation; and (b) filtering the first microorganism-containing concentrate to provide a permeate and a second microorganism-containing concentrate. In some additional embodiments, the process comprises the further step (c) recovering the second microorganism-containing concentrate. In some yet additional embodiments, the centrifuging is carried out at a centrifugation force from about 400 to about 65000 xg. In some alternative embodiments, the centrifuging is carried out at a centrifugation force from about 4000 to about 20000 xg.

In some yet additional embodiments, the filtering step comprises microfiltration. In some still further embodiments, the filtering step utilizes a filtration membrane having a pore size of about 0.1 to about 10 µm In some additional embodiments, the filtering step comprises ultrafiltration. In some still further embodiments, the utrafiltration step utilizes a filtration membrane having a molecular weight cut-off of about 5 to about 200 kDa. In some alternative embodiments, the filtering comprises tangential filtration.

In some additional embodiments, the centrifuging step is directly followed by the filtering step. In some further embodiments, the processes further comprise at least one additional filtering step, wherein the additional filtering step is performed on the microorganism-containing concentrate obtained after step (b).

In some yet additional embodiments, the processes further comprise at least one washing step. In some preferred embodiments, the washing step is not carried out between the centrifuging step (a) and the filtering step (b). In some alternative preferred embodiments, the washing step is carried out during and/or after the filtering step (b).

In some further embodiments, the processes further comprise the step of recovering the supernatant and/or permeate obtained after the centrifuging step and/or the filtering step.

In some preferred embodiments, the microorganism-containing suspension is a bacteria-containing suspension. In some particularly preferred embodiments, the bacteria of the bacteria-containing suspension comprise *Acetobacter*, *Bifidobacterium, Carnobacterium, Enterococcus, Lactococcus, Lactobacillus, Leuconostoc, Pediococcus, Oenococcus, Propionibacterium*, and/or *Streptococcus.* In yet additional preferred embodiments, the bacteria in the bacteria-containing suspension comprise at least one lactic acid bacteria genus. In some further preferred embodiments, the lactic acid bacteria comprises *Lactococcus, Lactobacillus, Leuconotoc, Carnobacterium, Pediococcus,* and/or *Streptococcus.* In some additional preferred embodiments, the lactic acid bacteria comprises at least one species comprising *Leuconostoc spp., Bifidobacterium ssp, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillu reuteri, Lactobacillus lactis, Lactobacillus delbreuckii, Lactobacillus plantarum,* and/or *Streptococcus thermophilus.*

The present invention also provides processes for concentrating a microorganism-containing suspension. It is also described microorganism-containing concentrates obtained by the processes set forth herein. In some preferred embodiments, the microorganism concentration is from about 10⁹ to about 10¹² cfu/mL.

### Description of the Figures

Fig.1 shows the relative cell count of a *Streptococcus thermophilus-*containing suspension and *Streptococcus thermophilus*-containing concentrates after the centrifugation step and each filtration step, as compared to the theoretical values.
Fig.2 shows the activity measurement of *Streptococcus thermophilus-*containing concentrates.
Fig.3 shows the cell counts of a *Lactobacillus acidophilus* concentrate.
Fig.4 shows the relative cell count of *Lactococcus lactis*-containing concentrates after centrifugation and filtration steps.
Fig. 5 shows the activity measurement of *Lactococcus lactis*-containing concentrates.

### Description of the Invention

The present invention provides processes for concentrating microorganism-containing suspensions. In some preferred embodiments, the processes comprise a centrifugation step followed by a filtration step. This process is particularly suitable for concentrating microorganism-containing suspensions (*e.g*., lactic acid bacteria suspensions) on an industrial scale.

US Pat No. 5,716,615 describes dietary and pharmaceutical compositions comprising lyophilized lactic bacteria at high concentrations per gram of product. The compositions are generally prepared at laboratory scale by centrifugation of a lactic bacteria containing composition. In one embodiment, the centrifugation step is preceded by micro filtration. However, the sequence of microfiltration followed by centrifugation is not feasible on the industrial scale when large volumes of suspensions need to be treated. Indeed as micro filtration is performed first, a large filtration area is required. Thus, the process is too expensive to use industrial scale. Moreover, on the industrial scale, the concentrate obtained after the microfiltration would also be difficult to further concentrate by centrifugation on the industrial scale, as the concentrate would be too thick. Finally, the final concentration rate would not be better than that obtained through use of a single centrifugation step.

The present invention provides methods to improve the concentration efficiency, productivity and cost associated with processing microbial suspensions. Indeed, these factors are essential parameters in industrial scale applications. Significantly, methods of the present invention provide an improved concentration rate, as well as limited loss of microbial activity. These methods utilize centrifugation and filtration.

In some embodiments, the present invention provides a process comprising the steps of:
(a) centrifuging a microorganism-containing suspension, to provide a first microorganism-containing concentrate and a supernatant liquid; and
(b) filtering the first microorganism-containing concentrate, to provide a permeate and a second microorganism-containing concentrate. In some preferred embodiments, the filtration step is a microfiltration or an ultrafiltration step.

In some embodiments, the centrifugation step (a) is carried out first and upon completion, is followed by the filtration step (b).

In some additional embodiments, the process is a continuous flow process comprising:
(a₁) centrifuging a microorganism-containing suspension to provide a first microorganism-containing concentrate and a supernatant liquid;
(a₂) continuously withdrawing the first microorganism-containing concentrate during the centrifugation step; and
(b) filtrating the first microorganism-containing concentrate to provide a permeate and a second microorganism-containing concentrate.

One advantage of the present invention is due to the order of the centrifugation and filtration steps. Due to this order, the process of the invention allows the rapid removal of large volumes of supernatant in the centrifugation step before proceeding to further concentrate the microbial suspension in the filtration step. This process is more efficient than methods known in the art, especially as applied to industrial scale processes. In addition, as a smaller filtration area is required for the methods of the present invention, cost savings are provided. Furthermore, the methods facilitate achievement of higher concentration ratios and less loss in microbial activity *(i.e.,* a smaller final suspension volume is provided with about the same level of microbial activity), as compared to methods that solely utilize centrifugation.

The methods of the present invention provide means for the rapid and efficient concentration of microorganism-containing suspensions. These methods provide for high concentration rates of microorganisms as well as limited loss of activity. Importantly, the methods of the present invention find use on an industrial scale, as significant volumes of microorganism-containing suspensions can be treated. In some embodiments, the methods of the present invention find use in treating suspensions of about 500 L to about 100,000 L. In some embodiments, the suspensions range from about 10,000 L to about 50,000 L, while in other embodiments, the range is from about 10,000 L to about 25,000 L. However, it is not intended that the present invention be limited to these volumes, as it is contemplated that any suitable volume of microorganism-containing suspension will find use in the methods of the present invention.

In addition, the present invention provides means by which a constant and/or predefined final concentration of microorganisms can be obtained. Thus, it is possible to produce final suspensions of microorganisms with a constant activity level. In some embodiments, the steps are modified, such that the desired concentration of microorganisms is obtained (e.g., by adjusting the flow rate through the filtration medium or membrane).

Furthermore, the methods of the present invention facilitate economical and efficient additional processing of the final suspensions. For example, because the water content of the final suspensions (*e.g*., the concentrate) is lower and the microorganism concentration is higher than suspensions produced using other methods, freeze-drying and lyophilisation of these suspensions is accomplished faster and more efficiently than using standard methods. Thus, by providing more rapid, efficient and economical means to preserve and store cultures, the present invention provides for reductions in energy, materials, and transportation costs.

As indicated above, using the methods of the present invention provide means to obtain the desired final concentration of microorganisms. The activity level of the microorganism suspensions is directly linked to the number of viable microorganisms. In some embodiments, the activity of the microorganisms (*i.e.,* the microbial activity level) is determined by assessing the amount of metabolite(s) the culture produces over a given time period and utilizing a specific type of substrate. For example, for lactic acid bacteria, it is possible to determine the activity level by continuously recording the pH for a given period of time, as the pH of a lactic acid bacterial suspension is directly linked to the concentration of viable bacteria. In some embodiments, comparing the recorded pH measurement to an expected theoretical pH value based on the assumption that all of the bacteria in the suspension are viable, provides the concentration and activity level of the suspension. Thus, if the measured pH is close to the theoretical value, the suspension has undergone limited activity loss during the process.

### Definitions

Unless defined otherwise herein, all technical and scientific terms used herein have the same meaning as commonly understood by one of ordinary skill in the art to which this invention pertains. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. It is to be understood that the present invention is not limited to the particular methodology, protocols, and reagents described, as these may vary, depending upon the context they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein. Furthermore, the citation of any document is not to be construed as an admission that it is prior art with respect to the present invention.

As used herein, the term "centrifugation" refers to a method of separating immiscible liquids or solids from liquids through application of centrifugal force. In some preferred embodiments, the separation methods involve subjecting a fluid-containing mixture to a high gravitational force (g). Upon application of this centrifugal force (often many times the force of gravity [xg]), the different components present in the mixture are separated. In some preferred embodiments, the present invention, centrifugation is applied to a liquid containing microbial cells. At the completion of the centrifugation process, the microbial cells are located in the "concentrate" (*i.e.,* the more "solid" portion of the product) and the liquid is the "supernatant" (or "eluate"). In some preferred embodiments, the liquid portion contains no or very few microorganisms. In industrial scale volumes, the concentrate typically contains from between about 5% and about 20% solids. In the methods of the present invention, centrifugation is used to produce the "first microorganism-containing concentrate."

In some preferred embodiments, centrifugation is carried out in a centrifuge that provides a gravitational force from about 400 to about 65000 xg *(i.e.,* times gravity), while in other embodiments, the gravitational force is from about 4000 to 20000 xg, and in other embodiments, the gravitational force is from about 6000 to about 10000 xg. However, it is not intended that the present invention be limited to any particular centrifuge or gravitational force. In some further embodiments of the present invention the centrifugation step is repeated between about two and about four times. In some particularly preferred embodiments, the centrifugation step is repeated twice. However, it is not intended that the present invention be limited to any particular number of repetitions of the centrifugation step, as any suitable number of repetitions will find use. In some embodiments, the concentrate obtained is placed in solution prior to each centrifugation step (*e.g.,* by adding any suitable liquid, such as water or food grade buffer). It is not intended that the present invention be limited to any particular liquid.

As used herein, the term "filtration" refers to a separation process consisting of passing a solution through a filtration membrane to separate the components within the liquid, based on the size of the components. The filtration membrane has pores of a given size designed to retain components that are larger than the pore size, but allow components that are smaller than the pore size to pass through the membrane. Thus, in some preferred embodiments, the solution contains solid elements (e.g., microorganisms) that are larger than the pores of the filtration membrane. In these embodiments, the microorganisms are present in the "concentrate" (or "retentate") and the liquid phase that passes through the membrane is referred to as "permeate" or "filtrate". In addition to containing liquid, in some embodiments, the permeate also contains other components. In the methods of the present invention, the filtration step results in the production of the "second microorganism-containing concentrate". In "conventional filtration" the separation is carried out due to natural gravitational pressure, while in "pressure filtration," additional pressure (*e.g*., greater pressure on the concentrate side and/or a depression on the permeate side) helps to accelerate the filtration process. Any suitable filtration methods find use in the present invention, including but not limited to microfiltration and ultrafiltration. However, in some particularly preferred embodiments, ultrafiltration is used.

As used herein, the term "microfiltration" refers to any filtration method that involves use of microporous filtration membranes. The pore size of these microfiltration membranes is usually about 0.1 µm to about 10 µm. The microfiltration membranes used in the methods of the present invention typically have a molecular weight cut-off of about 200 kDa to about 5 000 kDa.

As used herein, the term "ultrafiltration" refers to any filtration method using filtration membranes having smaller pore sizes than those used for microfiltration, usually about 0.01 µm to about 0.1 µm. The ultrafiltration membranes used in the methods of the present invention typically have a molecular weight cut-off of about 5 kDa to about 200 kDa.

In some methods of the present invention, the filtration step is accomplished using microfiltration with a microfiltration membrane having a pore size from about 0.1 to about 10 µm, while in other embodiments the pore size is from about 0.1 to about 5 µm, and in still other embodiments, the pore size is from about 0.1 to about 2 µm. In some preferred embodiments, the microfiltration membrane has a molecular weight cut-off of about 200 kDa to about 5 000 kDa, while in other embodiments, the molecular weight cut-off is about 250 kDa to about 1 000 kDa, and in still other embodiments, the molecular weight cut-off is about 500 kDa.

In some alternative methods of the present invention, the filtration step is accomplished using ultrafiltration with an ultrafiltration membrane having a pore size from about 0.01 µm to about 0.1 µm, while in other embodiments, the pore size is from about 0.02 to about 0.1 µm, and in still other embodiments, the pore size is from about 0.05 to about 0.1 µm. In some embodiments, the ultrafiltration membrane has a molecular weight cut-off of about 5 kDa to about 200 kDa, while in other embodiments, the molecular weight cut-off of is from about 30 kDa to about 200 kDa, and in still further embodiments, the molecular weight cut-off is about 150 kDa.

It is intended that any suitable filtration method will find use in the present invention, including but not limited to conventional filtration methods (*e.g*., by use of gravitational force) and tangential or cross-flow filtration methods. The term "cross flow filtration" and "tangential filtration" are used interchangeably herein in reference to any filtration method wherein the concentrate continuously and tangentially flows across the surface of the filtration membrane while the permeate flows trough the filtration membrane.

In some embodiments of the present invention, the process of concentrating microorganism-containing suspensions comprises at least one centrifugation step of a microorganism-containing suspension resulting in a first microorganism-containing concentrate and a supernatant, followed by an ultrafiltration step applied to the first microorganism-containing concentrate to produce a permeate and a second microorganism-containing concentrate. In some embodiments, the ultrafiltration step utilizes tangential ultrafiltration. In some embodiments, at least a portion of the microorganisms present in the first and/or second microorganism-containing concentrate are recovered. In some alternative embodiments, at least a portion of the microorganisms present in the first and/or second microorganism-containing concentrate are directly utilized in the production of food and/or other items (*e.g*., added to a milk mixture to produce yogurt).

Indeed, although any suitable filtration method finds use with the present invention, in some particularly preferred embodiments, ultrafiltration is used. As indicated herein, experiments showed that a smaller pore size facilitates faster concentration of the microorganisms. It was surprisingly found that some of the pores of the micro filtration membranes are fouled by microorganisms, thereby reducing the efficiency of micro filtration. As ultrafiltration membranes have a smaller pore size, this phenomenon does not occur during ultrafiltration. Thus, the efficiency of ultrafiltration is unaffected by this fouling. Ultrafiltration facilitates the maintenance of the flux *(i.e.,* the filtration rate) of the liquid through the filter.

In some embodiments, the methods of the present invention find use in batch processing, in which the centrifugation and filtration steps are conducted separately, while in other embodiments, the methods utilize "continuous, flow processing". In continuous flow processing, the centrifuge is continuously fed with the microorganism-containing suspension providing a continuous output flow of first microorganism-containing concentrate, which is then directly and continuously processed through the filtration step.

Thus, in some continuous flow process embodiments, the methods of the present invention comprise the steps of (a₁) centrifuging a microorganism-containing suspension to produce a first microorganism-containing concentrate and a supernatant liquid; (a₂) continuously withdrawing the first microorganism-containing concentrate during centrifugation; and (b) filtering the first microorganism-containing concentrate to produce a permeate and a second microorganism-containing concentrate. In some embodiments, the centrifugation step is directly followed by the filtration step.

In some additional embodiments, the methods further comprise at least one additional filtration step performed on the microorganism-containing concentrate obtained after step (b). Thus, in some embodiments, the microorganism-containing concentrate is subjected to several "cycles" of filtration. The goal is to provide a more highly concentrated concentrate *(i.e.,* the concentrate contains a higher concentration of microorganism). Any suitable filtration method finds use in this additional filtration step. Furthermore, this filtration step is suitable for as many repetitions as desired.

### Detailed Description of the Invention

The present invention provides processes for concentrating microorganism-containing suspensions. In some preferred embodiments, the processes comprise a centrifugation step followed by a filtration step. This process is particularly suitable for concentrating microorganism-containing suspensions (e.g., lactic acid bacteria suspensions) on an industrial scale.

The present invention provides processes for concentrating microorganism-containing suspensions. These methods comprise centrifugation of a microorganism-containing suspension to produce a first microorganism-containing concentrate and a supernatant, followed by filtration of the first microorganism-containing concentrate, to produce a permeate and a second microorganism-containing concentrate. In some particularly preferred embodiments, the centrifugation and filtration steps are followed by recovery of at least a portion the microorganisms from either or both the first and/or second microorganism-containing concentrate. In some further embodiments, at least a portion of the microorganisms in the first and/or second microorganism-containing concentrate are preserved using any suitable method known in the art (*e.g*., freezing, freeze drying or lyophilisation, etc.). In some additional embodiments, the second microorganism-containing concentrate is directly used in the production of a food or other product (*e.g.*, introducing the concentrate into a milk mixture to produce yogurt).

The first microorganism-containing concentrate contains the majority or essentially all of the microorganisms contained in the initial suspension. The filtration step serves to further concentrate the microorganisms in the first concentrate by removing liquid in the resultant filtrate.

In some embodiments, the methods of the present invention involve at least one washing step. Any suitable solution finds use in this washing step (*e.g.,* liquids such as water or food grade buffers). Utilization of this washing step further eliminates components contained in the microorganism-containing suspension(s). However, in some preferred embodiments, the washing step is not carried out between the centrifugation step(s) and the filtration step(s). in some particularly preferred embodiments, the washing step is carried out during and/or after the filtration step. In some embodiments, the washing step is carried out between additional filtration steps.

In some further embodiments, the supernatant obtained after the centrifugation step(s) and/or the permeate obtained after the filtration step(s) recovered. These embodiments find particular use when molecules of interest are present into the supernatant and/or the permeate. Some examples of molecules of interest include, but are not limited to bacteriocins, enzymes and lactic acid. In some embodiments, the supernatant obtained after the centrifugation step(s) and/or the permeate obtained after the filtration step(s) are subject to multiple centrifugation and/or filtration step(s) before the recovery of the molecule(s) of interest.

As indicated above, in some embodiments, the methods of the present invention further comprise preservation and/or storage steps. In some embodiments, freeze-drying, drying, freezing and/or cooling find use. Any suitable method finds use in the present invention. For example, drying can be accomplished using drum-drying, spray-drying, fluid bed drying, tray-drying, or any other suitable method. It is not intended that the present invention be limited to any particular preservation and/or storage steps.

The methods of the present invention find use in providing a highly concentrated suspension of any suitable microorganism, including but not limited to bacteria, viruses, fungi (*e.g*., molds and, yeasts), protozoans or algae.

In some embodiments, the microorganism-containing suspension is a fermentation broth containing bacteria. In some preferred embodiments, the bacteria comprise one or more of the genera *Acetobacter, Bifidobacterium, Carnobacterium, Enterococcus, Lactococcus, Lactobacillus, Leuconostoc, Pediococcus, Oenococcus, Propionibacteriurn* and *Streptococcus.* However, it is not intended that the present invention be limited to any particular genus or species of bacteria.

In some preferred embodiments, the microorganism-containing suspension, (*e.g.,* a fermentation broth), contains lactic acid bacteria. In some embodiments, the lactic acid bacteria are of at least one of the genera *Bifidobacterium, Lactococcus, Lactobacillus, Leuconostoc, Carnobacterium, Pediococcus,* and *Streptococcus.* In some particularly preferred embodiments, the lactic acid bacteria belong to at least one of the species of *Leuconostoc spp., Bifidobacterium ssp, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillu reuteri, Lactobacillus lactis, Lactobacillus delbreuckii, Lactobacillus plantarum* and *Streptococcus thermophilus.* However, it is not intended that the present invention be limited to any particular species, subspecies, strain, etc., of bacteria.

As indicated herein, the methods of the present invention are particularly useful and cost effective for concentrating microorganism-containing suspensions on an industrial scale, where large volumes of suspensions are treated (*e.g*., about 500 to about 100,000 L). In the methods of the present invention the centrifugation step considerably reduces the volume of solution, resulting in a first concentrate that is then further concentrated in the filtration step. Therefore, in some embodiments the process of the invention results in higher concentration rates of about 1.2 to about 10 times more efficient than centrifugation alone, while in other embodiments, the concentration rates are about 1.2 to about 5 times more efficient than centrifugation alone, and in still other embodiments, about 1.8 to about 2.5 times more efficient than centrifugation alone. Furthermore, the methods of the present invention tend to be less expensive, as compared to filtration alone, while the microbial activity is maintained through the process.

In some particularly preferred embodiments, the final microorganism-containing concentrate (*e.g*. the second microorganism-containing concentrate) is concentrated to an amount between about 22 times and about 80 times, compared to the concentration of the initial microorganism-containing suspension, while in other embodiments, the concentration range is between about 30 times and about 65 times, and in still other embodiments, the concentration range is between about 40 times and about 55 times compared to the microorganism concentration of the initial microorganism-containing suspension. Thus, in some embodiments, the final microorganism-containing suspension contains a high number of microorganisms per volume, at least about 10⁹ to about 10¹² cfu/mL, while in other embodiments, the final concentration is at least about 5.10⁹ to about 9.10¹¹ cfu/mL.

Thus, the process of present invention provides microorganism-containing concentrates. These concentrates find use in various applications, including, but not limited to food production, feed production, etc. As indicated above, the present invention finds use in providing concentrates of any suitable microorganism.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (revolutions per minute); xg (times gravity); H₂O (water); kD (kilodaltons); gm (grams); µg and ug (micrograms); mg (milligrams); ng (nanograms); µl and ul (microliters); ml (milliliters); mm (millimeters); nm (nanometers); µm and um (micrometer); M (molar); mM (millimolar); µM and uM (micromolar); kDa (kilodaltons); U (units); V (volts); MW (molecular weight); sec (seconds); min(s) (minute/minutes); hr(s) (hour/hours); CFU (colony-forming units); Alfa Laval (Alfa Laval, Richmond, VA); t, MI); GIBCO BRL or Gibco BRL (Life Technologies, Inc., Gaithersburg, MD); PTI Advanced Filtration (PTI Advanced Filtration Inc., Brooklyn Park, MN); Westfalia (Westfalia Separator, Inc., Northvale, NJ; TAMI (TAMI Industries, Nyons, FR); and Coors (Coors Ceramics, Denver, CO).

### Example 1

In this Example, a culture of *Streptococcus thermophilus,* grown in a standard medium containing milk solids, yeast extract and fermentable carbohydrate, under standard conditions, as known in the art was concentrated using the methods of the present invention.

First, 2500 L of the fermentation broth from the *Streptococcus thermophilus* culture were cooled down to 5-10°C and concentrated via centrifugation using an Alfa Laval disk stack centrifuge at a gravitational force of 8304 xg. The concentrate obtained was collected and maintained at 5-10°C.

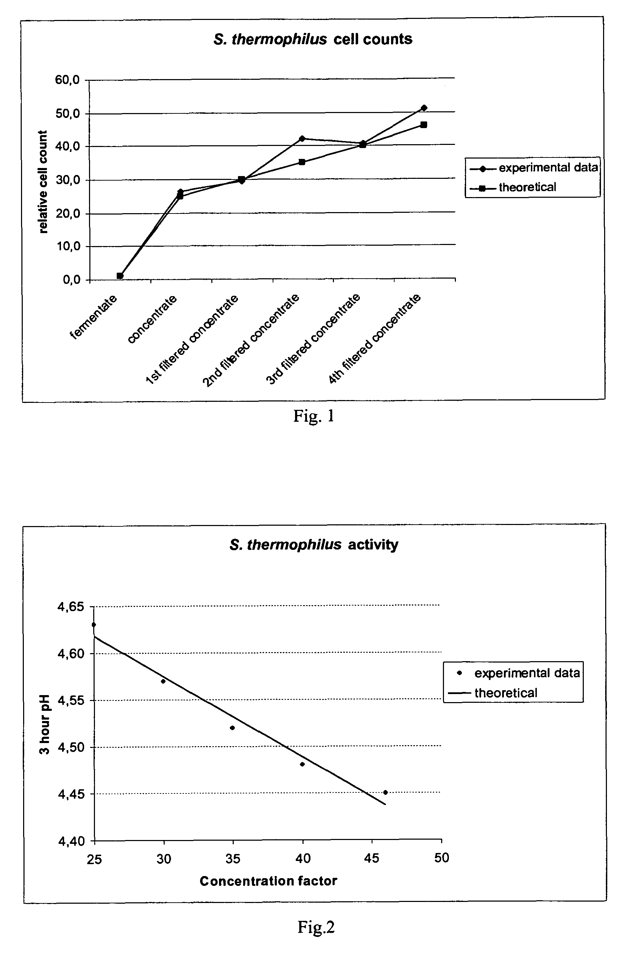

A 100kg batch of this concentrate obtained from the centrifugation step was concentrated using an Alfa Laval micro filtration system, with a polymeric membrane of pore size of 0.2 µm (Alfa Laval). The filtration step resulted in 45.8 kg of permeate (*i.e*., a concentration factor of 1.8x). However, the overall concentration factor was determined to be approximately 46x, as indicated in Table 1, below. Throughout the run, the collected permeate was crystal-clear, indicating that there was no passage of cells through the membrane. Samples of concentrate were taken throughout the run, and frozen for subsequent cell count and activity measurements. The samples were grown on M17 medium for 2 days at 37°C, as known in the art, before cell count measurements were taken. The results of these analyses are shown in Figures 1 and 2. Clearly, membrane filtration shows no deleterious effects on cell count or acidification activity of this strain of *Staphylococcus thermophilus.*

### Example 2

A culture of *Lactobacillus acidophilus* grown under standard conditions, in a standard medium containing milk solids, yeast extract and fermentable carbohydrate, as known in the art was concentrated using the methods of the invention.

First, 1600L of the fermentation broth of the *Lactobacillus acidophilus* strain were cooled down to 5-10°C, concentrated via centrifugation, and stored as described in Example 1.

Then, 72 kg of the concentrate obtained after centrifugation were concentrated using an ultrafiltration system (TAMI) with a ceramic membrane of pore size of 150kDa (TAMI). The filtration step produced 40 kgs of permeate, representing a concentration factor of 2.3x. Throughout the run, the collected permeate was crystal-clear, indicating no passage of cells through the membrane.

Samples of the concentrate were taken at the end of the run, and frozen for subsequent cell count and activity measurements.

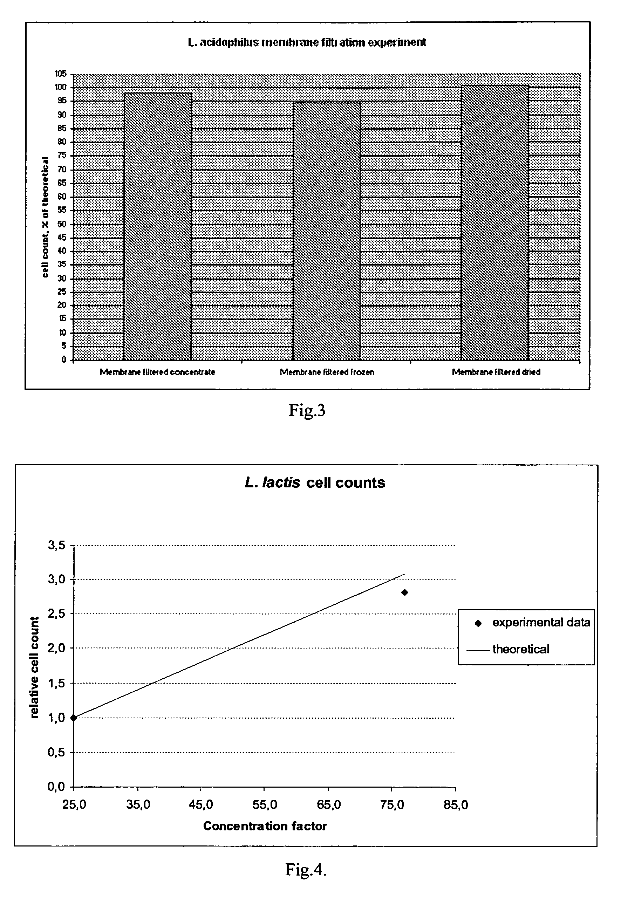

The samples were then grown on MAS medium for 2 days at 37°C, as known in the art, before cell count measurements were taken. The results of the cell count testing are shown in Figure 3. Clearly, membrane filtration resulted in no deleterious effects on cell count through all steps of the process for this *Lactobacillus acidophilus* culture as shown in Table 1, the overall concentration factor was 50x.

The concentrate obtained was then washed using water and subjected to an additional filtration step, as described above. The final overall concentration factor was determined to be 40x.

### Example 3

A culture of *Lactococcus lactis* was grown under standard conditions in a medium containing milk solids, yeast extract and fermentable carbohydrate, as known in the art, was concentrated using the methods of the present invention.

First, 2400L of the fermentation broth containing the *Lactococcus lactis* were cooled down to 5-10°C and concentrated via centrifugation on a Westfalia stack disk centrifuge, using a gravitational force of 9300 xg. The resulting concentrate was collected and stored at 5-10°C.

This concentrate was then further concentrated using a PTI microfiltration system, with a ceramic membrane of pore size of 0.2 µm (Coors). The resulting permeate was collected and measured, with a concentration factor of 3.1x. Throughout the run, the collected permeate was crystal-clear, indicating no passage of cells through the membrane.

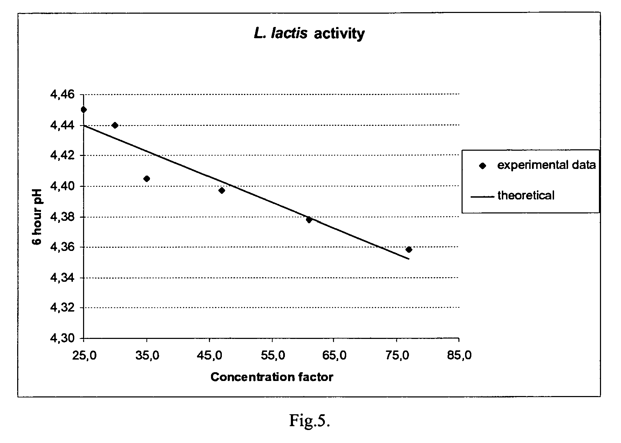

Samples of concentrate were taken throughout the run, and frozen for subsequent cell count and activity measurements. The samples were grown on MAS medium for 2 days at 30°C, as known in the art, before cell count measurements were taken. The results of these analyses are shown in Figures 4 and 5. The data from this experiment indicated that microfiltration resulted in no deleterious effects on cell count or acidification activity of this *Lactococcus lactis* culture.

| **Table 1. Experimental Results** | | | |
|---|---|---|---|
| **Strain** | **Concentration Factor for the Centrifugation Step** | **Concentration Factor for the Filtration Step** | **Approximate Overall Concentration Factor of the Full Process (Centrifugation Step (+ Filtration Step)** |
| *S. thermophilus* (Example 1) | 25x | 1.8x | 46x |
| *L. acidiphilus* (Example 2) | 22.2x | 2.3x | 50x |
| *L. lactis* (Example 3) | 25x | 3.1x | 77x |

## Claims

1. A process comprising the following steps:
(a) centrifuging a microorganism-containing suspension to provide a first microorganism-containing concentrate and a supernatant liquid;
(b) filtering said first microorganism-containing concentrate, to provide a permeate and a second microorganism-containing concentrate.

2. The process of claim 1, said process being a continuous flow process comprising:
(a₁) centrifuging a microorganism-containing suspension to provide a first microorganism-containing concentrate and a supernatant liquid;
(a₂) continuously withdrawing said first microorganism-containing concentrate during centrifugation; and
(b) filtering said first microorganism-containing concentrate to provide a permeate and a second microorganism-containing concentrate.

3. The process according to claim 1 or 2, further comprising step (c) consisting of recovering said second microorganism-containing concentrate.

4. The process according to any of claims 1 to 3, wherein said centrifuging step is carried out at a centrifugation force from 400 to 65000 xg, preferably from 4000 to 20000 xg.

5. The process according to any of claims 1 to 4, wherein said filtering step comprises microfiltration, preferably using a filtration membrane having a pore size of 0.1 to 10 µm.

6. The process according to any of claims 1 to 4, wherein said filtering step comprises ultrafiltration, preferably using a filtration membrane having a molecular weight cut-off of 5 to 200 kDa.

7. The process according to any of claims 1 to 6, wherein said filtering step comprises tangential filtration.

8. The process according to any of claims 1 to 7, wherein the centrifuging step is directly followed by the filtering step.

9. The process according to any of claims 1 to 8, further comprising at least one additional filtering step, wherein said additional filtering step is performed on the second microorganism-containing concentrate obtained in step (b).

10. The process according to any of claims 1 to 9, further comprising at least one washing step, said washing step preferably not being carried out between the centrifuging step (a) and the filtering step (b), and more preferably said washing step being carried out during and/or after the filtering step (b).

11. The process according to any of claims 1 to 10, further comprising the step of recovering the supernatant obtained in the centrifuging step and/or the permeate obtained in the filtering step.

12. The process according to any of claims 1 to 11, wherein said microorganism-containing suspension is a bacteria-containing suspension.

13. The process according to claim 12, wherein said bacteria-containing suspension comprises a bacteria selected from the group consisting of *Acetobacter, Bifidobacterium, Carnobacterium, Enterococcus, Lactococcus, Lactobacillus, Leuconostoc, Pediococcus, Oenococcus, Propionibacterium,* and *Streptococcus.*

14. The process according to claim 12, wherein said bacteria-containing suspension comprises at least one lactic acid bacteria genus, preferably selected from the group consisting of *Lactococcus, Lactobacillus, Leuconostoc, Carnobacterium, Pediococcus,* and *Streptococcus* and more preferably at least one specie selected from the group consisting of *Leuconostoc spp., Bifidobacterium ssp, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillus reuteri, Lactobacillus lactis, Lactobacillus delbreuckii, Lactobacillus plantarum,* and *Streptococcus thermophilus.*

## Patentansprüche

1. Verfahren umfassend die folgenden Schritte:
(a) Zentrifugieren einer Mikroorganismus-enthaltenden Suspension, um ein erstes Mikroorganismus-enthaltendes Konzentrat und eine Überstandsflüssigkeit bereitzustellen;
(b) Filtern des ersten Mikroorganismus-enthaltenden Konzentrats, um ein Permeat und ein zweites Mikroorganismus-enthaltendes Konzentrat bereitzustellen.

2. Verfahren gemäß Anspruch 1, wobei das Verfahren ein Continous-Flow-Verfahren ist, umfassend:
(a₁) Zentrifugieren einer Mikroorganismus-enthaltenden Suspension, um ein erstes Mikroorganismus-enthaltendes Konzentrat und eine Überstandsflüssigkeit bereitzustellen;
(a₂) kontinuierliches Entnehmen des ersten Mikroorganismus-enthaltenden Konzentrats während der Zentrifugation; und
(b) Filtern des ersten Mikroorganismus-enthaltenden Konzentrats, um ein Permeat und ein zweites Mikroorganismus-enthaltendes Konzentrat bereitzustellen.

3. Verfahren gemäß Anspruch 1 oder 2, ferner umfassend Schritt (c), bestehend aus der Gewinnung des zweiten Mikroorganismus-enthaltenden Konzentrats.

4. Verfahren gemäß einem der Ansprüche 1 bis 3, wobei der Zentrifugationsschritt bei einer Zentrifugationskraft von 400 bis 65000 xg, bevorzugt von 4000 bis 20000 xg, durchgeführt wird.

5. Verfahren gemäß einem der Ansprüche 1 bis 4, wobei der Filtrationsschritt Mikrofiltration umfasst, bevorzugt mittels einer Filtrationsmembranen mit einer Porengröße von 0,1 bis 10 µm.

6. Verfahren gemäß einem der Ansprüche 1 bis 4, wobei der Filtrationsschritt Ultrafiltration umfasst, bevorzugt mittels einer Filtrationsmembranen mit einer Molekulargewichtsgrenze von 5 bis 200 kDa.

7. Verfahren gemäß einem der Ansprüche 1 bis 6, wobei der Filtrationsschritt Tangential-Filtration umfasst.

8. Verfahren gemäß einem der Ansprüche 1 bis 7, wobei der Zentrifugationsschritt direkt von dem Filtrationsschritt gefolgt wird.

9. Verfahren gemäß einem der Ansprüche 1 bis 8, ferner umfassend zumindest einen zusätzlichen Filtrationsschritt, wobei der zusätzliche Filtrationsschritt an dem zweiten Mikroorganismus-enthaltenden Konzentrat erhalten in Schritt (b) durchgeführt wird.

10. Verfahren gemäß einem der Ansprüche 1 bis 9, ferner umfassend zumindest einen Waschschritt, wobei der Waschschritt bevorzugt nicht zwischen dem Zentrifugationsschritt (a) und dem Filtrationsschritt (b) durchgeführt wird, und mehr bevorzugt der Waschschritt während und/oder nach dem Filtrationsschritt (b) durchgeführt wird.

11. Verfahren gemäß einem der Ansprüche 1 bis 10, ferner umfassend den Schritt des Gewinnens des Überstandes erhalten in dem Zentrifugationsschritt und/oder des Permeats erhalten in dem Filtrationsschritt.

12. Verfahren gemäß einem der Ansprüche 1 bis 11, wobei die Mikroorganismus-enthaltende Suspension eine Bakterienenthaltende Suspension ist.

13. Verfahren gemäß Anspruch 12, wobei die Bakterienenthaltende Suspension ein Bakterium ausgewählt von der Gruppe bestehend aus *Acetobacter, Bifidobacterium, Carnobacterium, Enterococcus, Lactococcus, Lactobacillus, Leuconostoc, Pediococcus, Oenococcus, Propionibacterium,* und *Streptococcus* umfasst.

14. Verfahren gemäß Anspruch 12, wobei die Bakterienenthaltende Suspension zumindest eine Milchsäurebakteriengattung umfasst, bevorzugt ausgewählt von der Gruppe bestehend aus *Lactococcus, Lactobacillus, Leuconostoc, Carnobacterium, Pediococcus,* und *Streptococcus* und mehr bevorzugt zumindest eine Spezies ausgewählt von der Gruppe bestehend aus *Leuconostoc spp., Bifidobacterium ssp, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillus reuteri, Lactobacillus lactis, Lactobacillus delbreuckii, Lactobacillus plantarum,* und *Streptococcus thermophilus.*

## Revendications

1. Procédé comprenant les étapes suivantes :
(a) la centrifugation d'une suspension contenant des micro-organismes pour obtenir un premier concentré contenant des micro-organismes et un liquide surnageant ;
(b) la filtration dudit premier concentré contenant des micro-organismes pour obtenir un perméat et un second concentré contenant des micro-organismes.

2. Procédé selon la revendication 1, ledit procédé étant un procédé à écoulement continu comprenant :
(a₁) la centrifugation d'une suspension contenant des micro-organismes pour obtenir un premier concentré contenant des micro-organismes et un liquide surnageant ;
(a₂) le retrait continu dudit premier concentré contenant des microorganismes pendant la centrifugation ; et
(b) la filtration dudit premier concentré contenant des micro-organismes pour obtenir un perméat et un second concentré contenant des micro-organismes.

3. Procédé selon la revendication 1 ou 2, comprenant en outre l'étape (c) consistant en la récupération dudit second concentré contenant des micro-organismes.

4. Procédé selon l'une quelconque des revendications 1 à 3, dans lequel ladite étape de centrifugation est réalisée à une force de centrifugation de 400 à 65 000 xg, de préférence de 4000 à 20 000 xg.

5. Procédé selon l'une quelconque des revendications 1 à 4, dans lequel ladite étape de filtration comprend une microfiltration, de préférence au moyen d'une membrane de filtration ayant une taille de pore de 0,1 µm à 10 µm

6. Procédé selon l'une quelconque des revendications 1 à 4, dans lequel ladite étape de filtration comprend une ultrafiltration, de préférence au moyen d'une membrane de filtration présentant un seuil de coupure de poids moléculaire de 5 à 200 kDa.

7. Procédé selon l'une quelconque des revendications 1 à 6, dans lequel ladite étape de filtration comprend une filtration tangentielle.

8. Procédé selon l'une quelconque des revendications 1 à 7, dans lequel l'étape de centrifugation est suivie directement par l'étape de filtration.

9. Procédé selon l'une quelconque des revendications 1 à 8, comprenant en outre au moins une étape de filtration supplémentaire, dans lequel ladite étape de filtration supplémentaire est réalisée sur le second concentré contenant des microorganismes obtenu dans l'étape (b).

10. Procédé selon l'une quelconque des revendications 1 à 9, comprenant en outre au moins une étape de lavage, ladite étape de lavage n'étant pas réalisée de préférence entre l'étape (a) de centrifugation et l'étape (b) de filtration, et de manière davantage préférée ladite étape de lavage étant réalisée pendant et/ou après l'étape (b) de filtration.

11. Procédé selon l'une quelconque des revendications 1 à 10, comprenant en outre l'étape de récupération du surnageant obtenu dans l'étape de centrifugation et/ou du perméat obtenu dans l'étape de filtration.

12. Procédé selon l'une quelconque des revendications 1 à 11, dans lequel ladite suspension contenant des micro-organismes est une suspension contenant des bactéries.

13. Procédé selon la revendication 12, dans lequel ladite suspension contenant des bactéries comprend une bactérie choisie dans le groupe constitué de *Acetobacter, Bifidobacterium, Carnobacterium*, *Enterococcus*, *Lactococcus*, *Lactobacillus, Leuconostoc, Pediococcus, Oenococcus*, *Propionibacterium* et *Streptococcus.*

14. Procédé selon la revendication 12, dans lequel ladite suspension contenant des bactéries comprend au moins un genre de bactérie lactique, de préférence choisie dans le groupe constitué de *Lactococcus, Lactobacillus, Leuconostoc, Carnobacterium, Pediococcus* et *Streptococcus,* et de manière davantage préférée au moins une espèce choisie dans le groupe constitué de *Leuconostoc spp., Bifidobacterium ssp, Lactococcus lactis, Lactococcus cremoris, Lactobacillus acidophilus, Lactobacillus casei, Lactobacillus kefir, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sake, Lactobacillus reuteri, Lactobacillus lactis, Lactobacillus delbreuckii, Lactobacillus plantarum* et *Streptococcus thermophilus.*
